# EUROPEAN PATENT APPLICATION

(11) **EP 2 554 116 A1**
(43) Date of publication of application: **06.02.2013**
(21) Application number: 11762151.6
(22) Date of filing: 08.03.2011
(51) Int. Cl.: A61B 5/1459, A61B 5/00, A61B 5/1468

(54) **BODILY FLUID COMPONENT MEASUREMENT SYSTEM**

(30) Priority: 31.03.2010 JP 2010081405
(71) Applicant: Terumo Kabushiki Kaisha, Tokyo 151-0072 (JP); Olympus Corporation, Shibuya-ku Tokyo 151-0072 (JP)
(72) Inventor: KOHNO, Hiromasa, Ashigarakami-gun Kanagawa 259-0151 (JP); HAGI, Kouji, Ashigarakami-gun Kanagawa 259-0151 (JP); SUGIMOTO, Naoya, Tokyo 151-0072 (JP)
(74) Representative: TBK
(86) International application number: PCT/JP2011/001350
(87) International publication number: WO 2011/121897

(57) **Abstract**

The waterproof property of a bodily fluid component measurement system is improved. A bodily fluid component measurement system according to this invention includes a sensor unit (110) which is indwelled in the skin of a subject and connected to a bodily fluid in the subject to measure a predetermined bodily fluid component, and a transmission unit (120) which is detachably attached to the sensor unit (110) and transmits, to a display unit (130), a measurement signal obtained by the sensor unit (110) or a calculation result calculated as a concentration of the bodily fluid component in a bodily fluid of the same type as or different type from the bodily fluid based on the measurement signal. The sensor unit (110) measures the bodily fluid component and transmits the measurement signal to the transmission unit (120) by being activated by electromagnetic induction caused by an electromagnetic field generated by the transmission unit (120).

## Description

### TECHNICAL FIELD

The present invention relates to a bodily fluid component measurement system which continuously measures a bodily fluid in a subject and displays the concentration of a predetermined component contained in the bodily fluid and, more particularly, to a bodily fluid component measurement system which intermittently and continuously measures an interstitial fluid in a subject and displays a blood glucose level.

### BACKGROUND ART

Conventionally, a so-called SMBG (Self Monitoring of Blood Glucose) has been widely used, which measures blood with a blood glucose meter, taken from a fingertip or the like by puncturing it with a puncture instrument on the spot in order to measure and manage the blood glucose level of a diabetic patient by himself/herself.

Recently, in order to replace this, there has been developed a bodily fluid component measurement system used for CGM (Continuous Glucose Monitoring) which continuously monitors the blood glucose level of a patient with a dedicated sensor indwelled in the patient's skin or the like by puncturing it with a needle disposed on the sensor unit (see, for example, patent literature 1). This system has already been put into practice in Europe and the United States.

A bodily fluid component measurement system used for CGM includes a sensor unit which is always attached to a patient, a transmission unit which is mounted on the sensor unit and transmits a measurement signal obtained by the sensor unit to the outside, and a display device which calculates and displays a blood glucose level based on the signal transmitted from the transmission unit. This system is designed according to its specifications to replace the sensor unit with a new one at a frequency of about once for three to seven days.

For this reason, the sensor unit and the transmission unit are configured to be detachable from each other, and an electric contact for the supply of power and the exchange of measurement signals is disposed between the sensor unit and the transmission unit. The transmission unit supplies power to the sensor unit via the electric contact, and receives, via the electric contact, the measurement result obtained by the sensor unit using the supplied power.

When using the bodily fluid component measurement system having the above arrangement, the patient lives his/her daily life while the sensor unit on which the transmission unit is mounted is always attached to him/her. For this reason, it is important for the transmission unit and the sensor unit to be provided with a sufficient waterproof function, in order to allow the patient to take showers and baths.

### CITATION LIST

### PATENT LITERATURE

PTL1: Japanese PCT National Publication 2002-526137

### SUMMARY OF INVENTION

### TECHNICAL PROBLEM

It is, however, difficult to ensure a sufficient waterproof property in the arrangement in which the sensor unit and the transmission units which are configured to be detachable are connected to each other via the electric contact, as disclosed in patent literature 1.

This is because, even if the sensor unit and the transmission unit are designed to be waterproof to protect the devices inside the housings, it is difficult to protect the electric contact exposed to the housing surfaces against water immersion.

In contrast to this, it is possible to prevent the exposed electric contact from being immersed with water, by entirely covering the sensor unit and the transmission unit with a waterproof sheet or the like from outside. It is, however, difficult to perfectly hermetically seal the sensor unit and the transmission unit. When, for example, the user takes a bath, the sensor unit and the transmission unit are soaked with water. In addition, bonding a waterproof sheet to the transmission unit makes it impossible to easily replace the transmission unit. For this reason, when using the sensor unit for a long period of time, it is necessary to use a power supply having a corresponding capacity. This leads to an increase in the size of the transmission unit, resulting in deterioration in wearing sensation.

The present invention has been made in consideration of the above problems, and has as its object to improve the waterproof property of a bodily fluid component measurement system and to increase the during of use of the sensor unit, reduce the size of the transmission unit, and improve a sense of fitting.

### SOLUTION TO PROBLEM

In order to achieve the above object, a bodily fluid component measurement system according to the present invention includes the following arrangement. That is,
this system is characterized by comprising:
a sensor unit which is indwelled in the skin of a subject and connected to a bodily fluid in the subject to measure a predetermined bodily fluid component; and
a transmission unit which is detachably attached to the sensor unit and transmits, to a display device, a measurement signal obtained by the sensor unit or a calculation result calculated as a concentration of the bodily fluid component in a bodily fluid of the same type as or different type from the bodily fluid based on the measurement signal,
characterized in that the sensor unit measures the bodily fluid component and transmits the measurement signal to the transmission unit by being activated by electromagnetic induction caused by an electromagnetic field generated by the transmission unit.

### ADVANTAGEOUS EFFECTS OF INVENTION

According to the present invention, since the bodily fluid component measurement system has no electric contact between the sensor unit and the transmission unit, the waterproof property improves. In addition, since it is easy to attach and detach the transmission unit including the power supply, detaching the transmission unit will prevent it from interfering with activity such as taking a bath, thereby improving the quality of life. Furthermore, since it is easy to recharge or replace the transmission unit upon detaching it, there is no need to increase the size of the transmission unit when using it for a long period of time.

Other features and advantages of the present invention will be apparent from the following description taken in conjunction with the accompanying drawings. Note that the same reference numerals denote the same or like components throughout the accompanying drawings.

### BRIEF DESCRIPTION OF DRAWINGS

The accompanying drawings are included in the specification, constitute a part of the specification, illustrate the embodiments of the present invention, and are used to explain the principle of the present invention together with the description.
Fig. 1 shows the outer arrangement of a bodily fluid component measurement system according to an embodiment of the present invention;
Fig. 2 is a block diagram showing the functional arrangement of a bodily fluid component measurement system 100 according to the first embodiment of the present invention;
Fig. 3 is a flowchart showing a procedure for monitoring processing in the bodily fluid component measurement system 100;
Fig. 4 is a flowchart showing a procedure for calibration value data recording processing in the bodily fluid component measurement system 100;
Fig. 5 is a flowchart showing a procedure for measurement processing in the sensor unit and transmission unit of the bodily fluid component measurement system 100;
Fig. 6 shows the electromagnetic field generation timing of the transmission unit of the bodily fluid component measurement system 100 and a change in the remaining amount of a secondary battery in the transmission unit;
Fig. 7 is a block diagram showing the functional arrangement of a bodily fluid component measurement system 700 according to the second embodiment of the present invention;
Fig. 8 is a flowchart showing a procedure for measurement processing in the sensor unit and transmission unit of the bodily fluid component measurement system 700;
Fig. 9 shows the electromagnetic field generation timing of the transmission unit of the bodily fluid component measurement system 700, a change in the remaining amount of a secondary battery in the transmission unit, and a change in the remaining amount of a battery in the sensor unit;
Fig. 10 is a block diagram showing the functional arrangement of a bodily fluid component measurement system 1000 according to the third embodiment of the present invention;
Fig. 11A is a flowchart showing a procedure for measurement processing in the sensor unit and transmission unit of the bodily fluid component measurement system 1000;
Fig. 11B is a flowchart showing a procedure for measurement processing in the sensor unit and transmission unit of the bodily fluid component measurement system 1000;
Fig. 12 shows the electromagnetic field generation timing of the transmission unit of the bodily fluid component measurement system 1000, a change in the remaining amount of a secondary battery in the transmission unit, and a change in the remaining amount of a secondary battery in the sensor unit;
Fig. 13A is a flowchart showing a procedure for measurement processing in the sensor unit and transmission unit of a bodily fluid component measurement system 1300;
Fig. 13B is a flowchart showing a procedure for measurement processing in the sensor unit and transmission unit of the bodily fluid component measurement system 1300;
Fig. 14 shows the electromagnetic field generation timing of the transmission unit of the bodily fluid component measurement system 1300, a change in the remaining amount of a secondary battery in the transmission unit, and a change in the remaining amount of a capacitor in the sensor unit;
Fig. 15 is a block diagram showing the functional arrangement of a bodily fluid component measurement system 1500 according to the fifth embodiment of the present invention;
Fig. 16 shows the outer arrangement of a sensor unit 110; and
Fig. 17 shows the outer arrangement of the sensor unit 110.

### DESCRIPTION OF EMBODIMENTS

Each embodiment of the present invention will be described in detail below with reference to the accompanying drawings, as needed. Note that the present invention is not limited to the following embodiments and can be modified, as needed.

### [First Embodiment]

Each embodiment of the present invention will be described below with reference to the accompanying drawings.

### <1. Outer Arrangement of Bodily Fluid Component Measurement System>

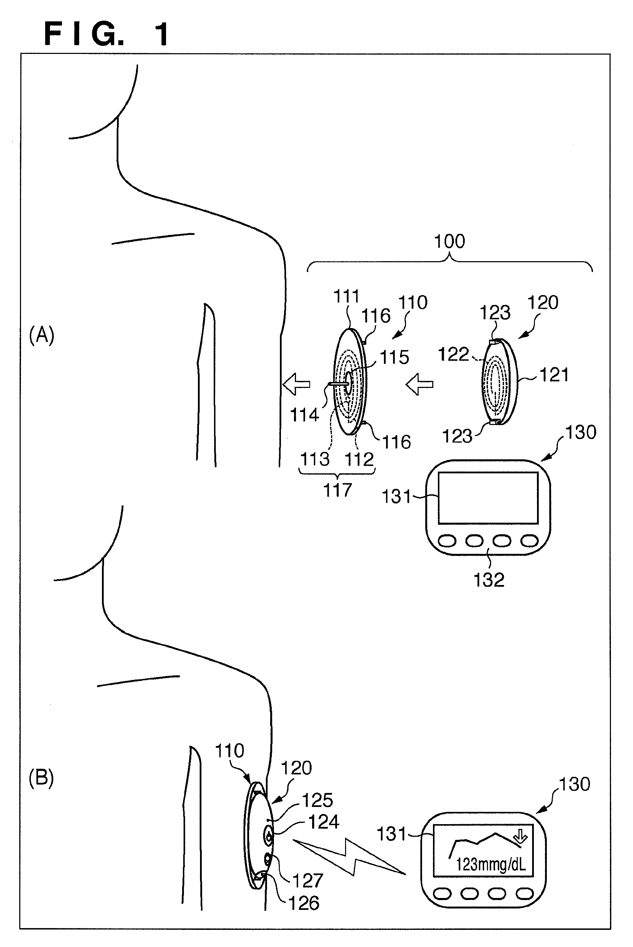

Fig. 1 is a view showing the outer arrangement of a bodily fluid component measurement system 100 according to an embodiment of the present invention.

As indicated by (A) in Fig. 1, the bodily fluid component measurement system 100 includes a sensor unit 110, a transmission unit 120, and a display unit 130.

The sensor unit 110 includes a main body unit 111 formed into a liquid-tight structure made of a flexible resin such as polyurethane. The bottom surface of the main body unit 111 is provided with a waterproof skin tape (not shown). With this tape, the sensor unit 110 is directly pasted on the brachial part or abdominal part of the subject.

The bottom surface of the main body unit 111 is also provided with a needle 114 punctured in the skin of the subject and comes into contact with a bodily fluid under the skin. The needle 114 is connected to a component measurement unit 115 disposed in the main body unit 111. The component measurement unit 115 measures a signal corresponding to the amount of a predetermined component such as glucose in the bodily fluid under the skin with which the needle 114 comes into contact.

The main body unit 111 is internally provided with an A/D circuit built-in IC tag 117 (to be referred to as an IC tag 117 hereinafter) including an antenna 112, an IC chip 113, and an A/D conversion circuit (not shown). The main body unit 111 operates on the electromotive force, as power, generated by the electromagnetic field generated in the transmission unit 120 to control measurement by the component measurement unit 115. The main body unit 111 also stores a measurement result in a memory in the IC chip 113 or transmits the result to the transmission unit 120.

The rear surface of the main body unit 111 is provided a lock portion 116. This can lock the transmission unit 120 to the rear surface side.

The transmission unit 120 includes a housing 121. The housing 121 is internally provided with an IC tag transmission/reception module 122 which is driven to generate an electromagnetic field for the IC tag 117 of the sensor unit 110, supplies power by electromagnetic induction, and receives a measurement signal representing a measurement result by detecting a change in the electromagnetic field generated by the IC tag 117. Assume that the transmission unit 120 is configured to process the measurement signal received from the sensor unit 110, calculate the concentration of a predetermined component (the concentration of a bodily fluid component) contained in a bodily fluid, such as a blood glucose level, and then transmit the calculated concentration of the bodily fluid component to the display unit 130 capable of communication via a transmission/reception module (not shown).

The bottom surface of the housing 121 is provided with a lock portion 123. The lock portion 123 is locked to the lock portion 116 on the rear surface of the main body unit 111 to mount the transmission unit 120 on the sensor unit 110.

The display unit 130 includes a display area 131, and displays the concentration of the bodily fluid component transmitted by the transmission unit 120. The display unit 130 also includes an input unit 132. The user uses the input unit 132 to perform various kinds of operations such as switching between the display contents displayed on the display area 131 and inputting predetermined information.

As described above, this system is configured to make the transmission unit 120 supply power and make the sensor unit 110 transmit a measurement signal between the sensor unit 110 and the transmission unit 120, wirelessly, by using the IC tag 117 and the IC tag transmission/reception module 122. This makes it unnecessary to expose any electric contact on the surface of the sensor unit 110, and can easily implement the liquid-tight structure of the main body unit 111. It is therefore possible to improve the waterproof property of the sensor unit 110.

In addition, since it is not necessary to connect the sensor unit 110 to the transmission unit 120 via an electric contact when supplying power and transmitting a measurement signal between them, there is no need to air-tightly mount the transmission unit 120 on the sensor unit 110 at a predetermined connecting position. This makes it possible to mount the transmission unit 120 on the sensor unit 110 with simple lock portion.

This makes it easy to attach and detach the transmission unit 120 to and from the sensor unit 110, and allows the subject to easily attach and detach the transmission unit 120. When taking a shower or bath, therefore, the subject can use this system upon easily detaching the transmission unit 120. This makes it unnecessary to always provide a waterproof structure for the transmission unit 120. That is, it is possible to obtain an extra effect of reducing the cost of the transmission unit 120.

In Fig. 1, (B) indicates how the transmission unit 120 is mounted on the sensor unit 110. As indicated by (B) in Fig. 1, the rear surface of the transmission unit 120 is provided with a power switch 124 for turning on/off the power supply of the transmission unit 120. The power switch 124 turns on the power supply to turn on a lamp 125 when being pressed once. This switch turns off the power supply to turn off the lamp 125 when being pressed again.

The rear surface of the transmission unit 120 is further provided with a speaker 127. When, for example, an error or the like is detected in the transmission unit 120, the speaker 127 outputs a sound to the subject. In addition, the rear surface of the transmission unit 120 is provided with a recharging connector 126. Connecting the recharging connector 126 to a recharging adaptor can recharge the secondary battery built in the transmission unit 120.

In the above arrangement, when the subject presses the power switch 124 to turn on the power supply of the transmission unit 120 while the transmission unit 120 is mounted on the sensor unit 110, the display unit 130 displays the concentration of a predetermined bodily fluid component every predetermined period (the display unit 130 in (B) in Fig. 1 displays, in the display area 131, the concentration of a bodily fluid component obtained by the current measurement together with a trend graph).

### <2. Functional Arrangement of Bodily Fluid Component Measurement System>

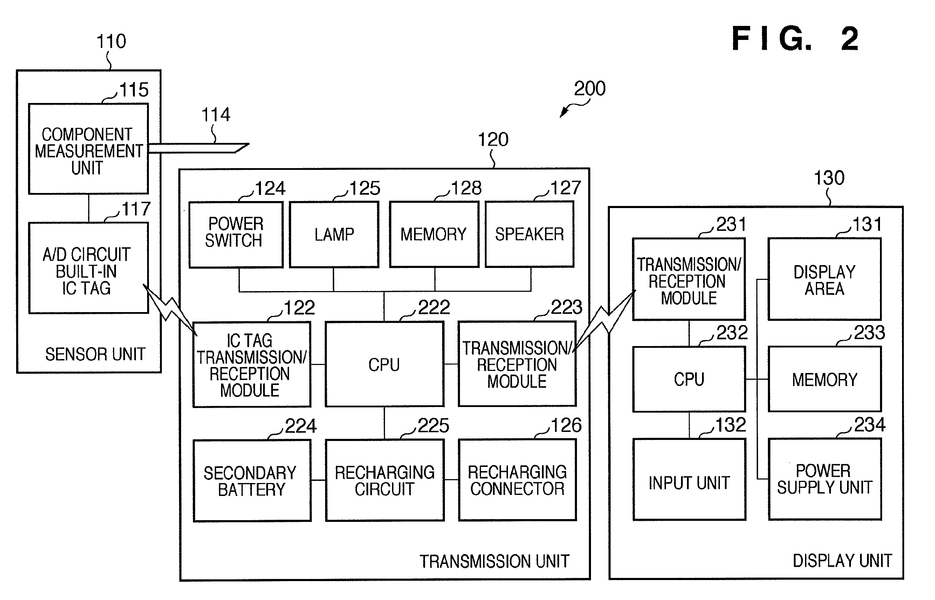

The functional arrangement of the bodily fluid component measurement system 100 will be described next. Fig. 2 is a block diagram showing the functional arrangement of the bodily fluid component measurement system 100. Note that the same reference numerals as in Fig. 2 denote elements corresponding to the elements described with reference to the outer arrangement in Fig. 1.

As shown in Fig. 2, the sensor unit 110 includes the needle 114, the component measurement unit 115, and the IC tag 117. The needle 114 is a capillary tube which guides a bodily fluid to the component measurement unit 115, and has a length that makes its distal end reach a position under the skin. The needle 114 is connected to the component measurement unit 115 and guides a bodily fluid into the component measurement unit 115.

The component measurement unit 115 is a measurement means for measuring the concentration of a specific component (for example, glucose, uric acid, cholesterol, protein, mineral, or blood cell) contained in a bodily fluid such as blood. The component measurement unit 115 performs measurement by using a known measurement method. The bodily fluid conducted by the needle 114 includes blood, interstitial fluid, or lymph fluid. The measurement method used includes a method of measuring the intensity of fluorescence generated upon application of exciting light to a fluorescent dye which captures a predetermined component contained in a bodily fluid in a measurement target and a method of optically or electrochemically measuring a predetermined component contained in a bodily fluid in a measurement target by using an oxidative enzyme. In this embodiment, the needle 114 is inserted under the skin to measure a glucose concentration in an interstitial fluid, and the measured concentration is converted into a glucose concentration in blood (blood glucose level). Assume that the embodiment uses a measurement method using a fluorescence sensor obtained by immobilizing, to a hydrogel, a boronic acid group doped fluorescent dye which binds to a sugar group to indicate a Stokes shift. Note that it is also possible to directly measure a glucose concentration in blood by inserting the needle 114 into a blood vessel instead of a subcutaneous region.

The IC tag 117 controls the component measurement unit 115 by using the electromotive force generated by the electromagnetic field generated in the transmission unit 120. The IC tag 117 also transmits, to the transmission unit 120, a digital measurement signal obtained by A/D-converting the measurement result from the component measurement unit 115.

The transmission unit 120 includes the power switch 124, the lamp 125, a memory 128, the speaker 127, the IC tag transmission/reception module 122, a CPU 222, a transmission/reception module 223, a secondary battery 224, a recharging circuit 225, and the recharging connector 126.

When the power switch 124 is pressed, the power supply of the transmission unit 120 is turned on to turn on the lamp 125. Note that when the remaining amount of the secondary battery 224 becomes small, it is also possible to blink the lamp 125 to notify the user that the remaining amount of the secondary battery 224 is small. In addition, the lamp 125 may be configured to blink in different colors in accordance with the internal states of the transmission unit 120 (for example, it blinks in green when the internal state is normal, and in red upon detection of an abnormality).

The memory 128 stores calculation programs each used to calculate the concentration of a predetermined component contained in a bodily fluid based on the digital measurement signal as the measurement result received from the sensor unit 110, calibration programs each used to calculate calibration value data used when calculating the concentration of a bodily fluid component, a control program used to control the overall operation of the transmission unit 120, and the like.

Assume that the calibration programs and the calculation programs are prepared for the respective bodily fluids as measurement targets and the respective components as calculation target. This allows the transmission unit 120 to calculate concentrations of various bodily fluid components. In addition, the memory 128 includes a function as a storage means for storing the concentrations of bodily fluid components calculated in the past in correspondence with the measurement times.

The speaker 127 notifies the end of measurement, the concentration of a bodily fluid component measured, and the like with sounds or the like. If, for example, a blood glucose level obtained as a result of measurement is normal, the speaker 127 continuously outputs a short beep sound, whereas if the blood glucose level is abnormal, the speaker 127 outputs a warning sound louder than the short beep sound.

The IC tag transmission/reception module 122 supplies power and receives a measurement signal between the transmission unit 120 and the sensor unit 110. The CPU 222 controls the overall operation of the transmission unit 120. The transmission/reception module 223 is a communication module which transmits the concentration of a bodily fluid component calculated by the transmission unit 120 to the display unit 130.

The secondary battery 224 supplies power to each unit constituting the transmission unit 120. The recharging circuit 225 is a circuit for recharging the secondary battery 224. The recharging circuit 225 recharges the secondary battery 224 when receiving power from an adaptor (not shown) connected via the recharging connector 126.

The display unit 130 includes a transmission/reception module 231, a CPU 232, the input unit 132, the display area 131, a memory 233, and a power supply unit 234.

The transmission/reception module 231 receives the concentration of a bodily fluid component transmitted from the transmission unit 120 via the transmission/reception module 223. The CPU 232 processes the concentration of the bodily fluid component received by the transmission/reception module 231. The CPU 232 then displays the resultant information in the display area 131 and stores it in the memory 233.

The input unit 132 includes buttons which accept an input instruction from the subject. The input unit 132 is used to issue an instruction to turn on the power supply of the display unit 130, an instruction to call up information concerning the concentration of a bodily fluid component calculated in the past, and an instruction to perform display switching and is also used to perform operation such as inputting information for the calculation of calibration value data used to calculate the concentration of a bodily fluid component. Note that the display area 131 and the input unit 132 may be formed from one component such as a touch panel. The power supply unit 234 is a battery for supplying power to each unit constituting the display unit 130.

### <3. Monitoring Processing in Bodily Fluid Component Measurement System>

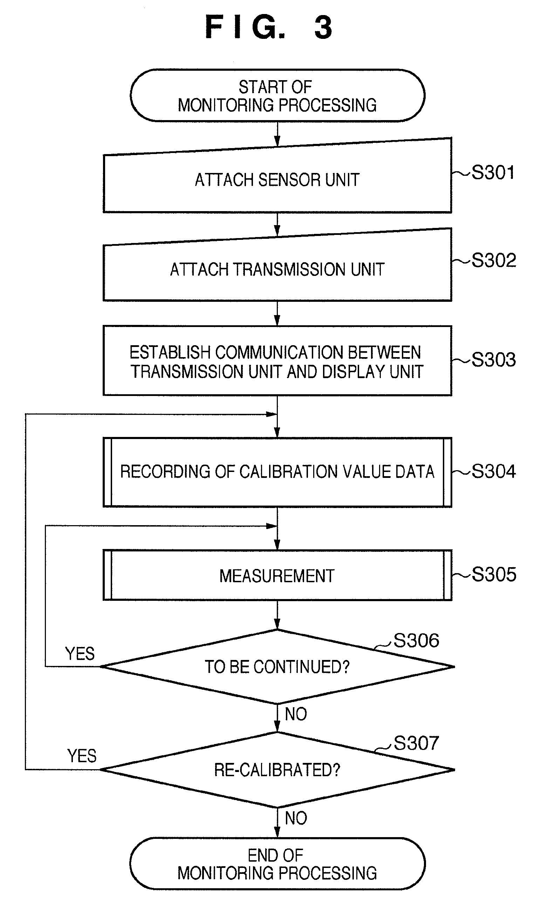

A procedure for monitoring processing in the bodily fluid component measurement system 100 will be described next. Fig. 3 is a flowchart showing a procedure for monitoring processing in the bodily fluid component measurement system 100. For the sake of simplicity, in the following description, assume that bodily fluid as measurement target = interstitial fluid and component as concentration calculation target = blood glucose level (that is, a blood glucose level is to be calculated as the concentration of a bodily fluid component).

In step S301, the subject wears the sensor unit 110 on his/her brachial portion or abdominal portion. More specifically, the subject punctures the skin of his/her brachial portion or abdominal portion with the needle 114, and brings the main body unit 111 into tight contact with the skin by using the waterproof skin tape with the waterproof function disposed on the bottom surface of the main body unit 111.

In step S302, the subject mounts the transmission unit 120 on the rear surface of the sensor unit 110 attached to the brachial portion or abdominal portion. More specifically, the subject locks the lock portion 123 provided on the bottom surface of the transmission unit 120 to the lock portion 116 provided on the rear surface of the sensor unit 110.

In step S303, the subject presses the power switch 124 of the transmission unit 120 to turn on the power supply of the transmission unit 120. This activates the transmission unit 120 to establish communication between the transmission unit 120 and the display unit 130.

In step S304, the system records the calculated calibration value data in the memory 128. Recording processing of calibration value data (step S304) will be described in detail later.

In step S305, the system performs measurement processing of performing measurement every predetermined period and displaying a blood glucose level. When the system completes measurement processing once, the process advances to step S306 to determine whether to continue monitoring processing. If the system determines in step S306 that it continues monitoring processing, the process returns to step S305 to perform measurement after a predetermined period and displays a blood glucose level. If the system determines in step S306 not to continue monitoring processing, the process advances to step S307.

In step S307, the system determines whether to perform re-calibration. If the system determines that it performs re-calibration, the process returns to step S304 to perform recording processing of the calibration value data. Upon determining that it does not perform re-calibration, the system terminates the monitoring processing.

### <4. Calibration Value Data Recording Processing>

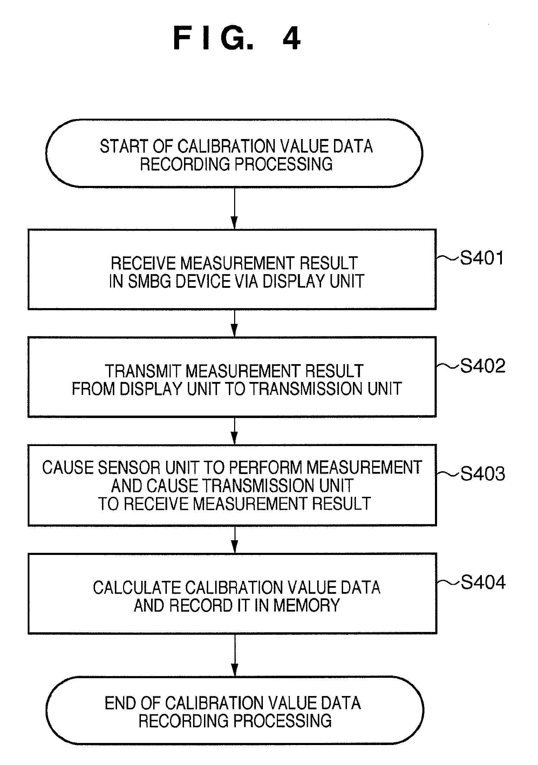

Calibration value data recording processing (step S304) will be described in detail next. Fig. 4 is a flowchart showing the details of a procedure for calibration value data recording processing (step S304).

In step S401, the subject inputs the blood glucose level obtained by measurement by SMBG (Self Monitoring of Blood Glucose) via the display unit 130. When the display unit 130 accepts the input, the transmission/reception module 231 transmits the input blood glucose level to the transmission unit 120 in step S402.

When the transmission unit 120 receives the blood glucose level transmitted by the display unit 130, the IC tag transmission/reception module 122 is activated and generates an electromagnetic field to the sensor unit 110 in step S403. This activates the sensor unit 110 and causes the component measurement unit 115 to measure a signal corresponding to the glucose amount in the interstitial fluid. Since the measurement result is transmitted to the transmission unit 120 via the IC tag 117, the transmission unit 120 receives it.

In step S404, the transmission unit 120 activates the calibration program to calculate calibration value data for a calculation expression for calculating a blood glucose level based on the blood glucose level transmitted by the display unit 130 in step S401 and the measurement result transmitted by the sensor unit 110 in step S403. The system then records the calculated calibration value data in the memory 128.

### <5. Measurement Processing>

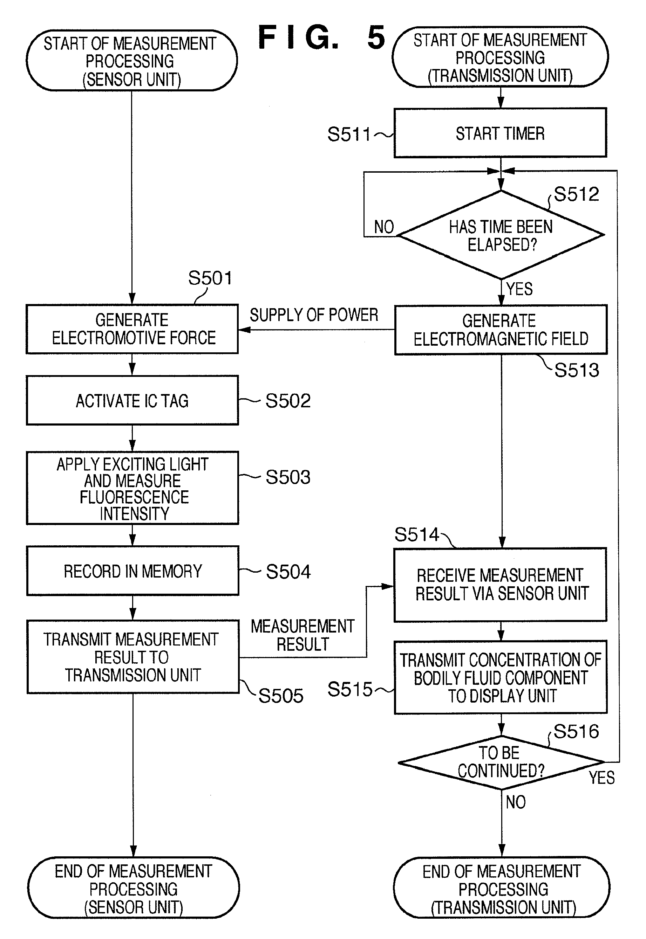

Measurement processing (step S305) will be described in detail next. Fig. 5 is a flowchart showing the details of a procedure for measurement processing (step S305).

When the transmission unit 120 starts measurement processing, the system starts a timer for defining a measurement interval for performing measurement at a predetermined period in step S511. In step S512, the system determines whether the time has counted a predetermined time. If the system determines in step S512 that the timer has counted the predetermined time, the process advances to step S513.

In step S513, the system drives the IC tag transmission/reception module 122 to generate an electromagnetic field. In step S501, when the IC tag transmission/reception module 122 generates an electromagnetic field, the IC tag 117 generates an electromotive force (that is, the transmission unit 120 supplies power to the sensor unit 110).

Upon supply of power in step S501, the system activates the IC tag 117 to supply power to the component measurement unit 115 and activate the component measurement unit 115 in step S502.

In step S503, the interstitial fluid sucked by the needle 114 has reached the hydrogel having a sugar-inducible fluorescent dye, and the component measurement unit 115 applies exciting light to the hydrogel. In addition, the component measurement unit 115 measures a fluorescence intensity corresponding to the applied exciting light.

In step S504, the system converts the fluorescence intensity measured in step S503 into a digital signal and records it as a measurement result in the memory in the IC tag 117. In step S505, the IC tag 117 transmits the measurement signal, which has been converted into a digital signal indicating the fluorescence intensity, to the IC tag transmission/reception module 122.

In step S514, upon receiving the measurement signal transmitted by the sensor unit 110, the transmission unit 120 reads out a calculation expression and calibration value data recorded in the memory 128 in advance, and calculates a blood glucose level from the received measurement signal by using the readout calculation expression and calibration value data.

In step S515, the system transmits the blood glucose level calculated in step S514 to the display unit 130. In step S516, the system determines whether to continue the measurement processing. Note that this embodiment is configured such that in steps S514 and S515, the transmission unit 120 calculates a blood glucose level and transmits it to the display unit 130. However, the embodiment may be configured such that the transmission unit 120 transmits a measurement signal to the display unit 130, and the display unit 130 calculates a blood glucose level based on the measurement signal.

If the system determines in step S516 that it continues the measurement processing, the process returns to step S512. If the system determines in step S516 that it does not continue the measurement processing, the system terminates the measurement processing.

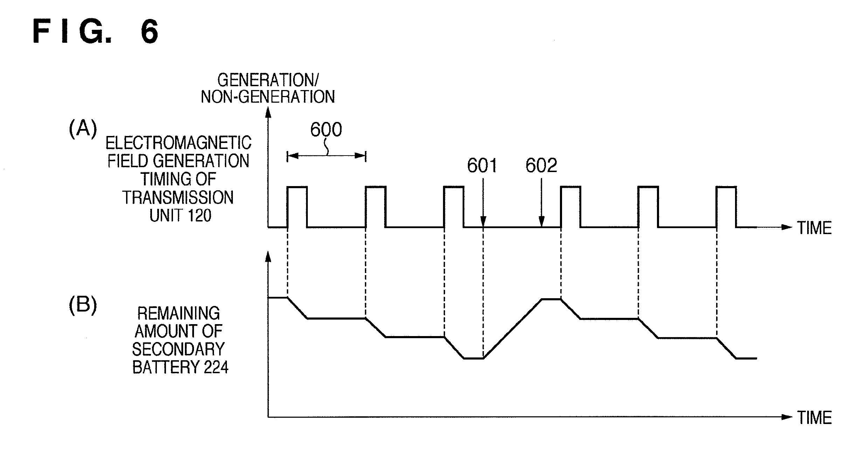

<6. Electromagnetic Field Generation Timing of Transmission Unit and Change in Remaining Amount of Secondary Battery>
The electromagnetic field generation timing of the transmission unit 120 and a change in the remaining amount of the secondary battery 224 at the time of measurement processing in the bodily fluid component measurement system 100 will be described next. Fig. 6 shows the electromagnetic field generation timing of the transmission unit 120 and a change in the remaining amount of the secondary battery 224 in measurement processing in the bodily fluid component measurement system 100.

As shown in Fig. 6, when measurement processing starts, the IC tag transmission/reception module 122 generates an electromagnetic field every predetermined period 600, and the component measurement unit 115 performs measurement. As a result, the remaining amount of the secondary battery 224 decreases every predetermined period 600.

Assume that after measurement was executed a plurality of number of times, the subject has temporarily detached the transmission unit 120 from the sensor unit 110 and performed recharging operation to take a shower or bath (timing 601).

With this operation, the remaining amount of the secondary battery 224 in the transmission unit 120 increases, and recharging completes at a timing 602. Subsequently, when the subject attaches the transmission unit 120 to the sensor unit 110 again and turns on the power supply, measurement processing starts again. The IC tag transmission/reception module 122 generates an electromagnetic field again every predetermined period 600, and the component measurement unit 115 performs measurement. As a result, the remaining amount of the secondary battery 224 decreases every predetermined period 600.

As described above, in the bodily fluid component measurement system 100, the sensor unit 110, which is always attached to the subject, incorporates no battery, and power is supplied to the sensor unit 110 by using an electromagnetic field from the transmission unit 120. For this reason, the remaining amount of the secondary battery 224 decreases in accordance with the electromagnetic field generation timing of the transmission unit 120. However, since the transmission unit 120 can be easily detached from the sensor unit 110 and is configured to be rechargeable, it is possible to easily recharge the transmission unit 120. That is, this system is configured to be suitable for continuously monitoring the concentration of a bodily fluid component.

As obvious from the above description, the bodily fluid component measurement system 100 according to this embodiment is configured to perform supply of power and transmission of measurement signals between the sensor unit 110 and the transmission unit 120 by using the IC tag 117 and the IC tag transmission/reception module 122.

This eliminates the necessity to expose an electric contact on the surface of the sensor unit 110, and hence makes it possible to easily implement the liquid-tight structure of the main body unit 111 and facilitate attachment/detachment of the transmission unit 120. As a consequence, it is possible to improves the waterproof property of the sensor unit 110 and achieve improvement in operability and a sense of fitting.

The unnecessity to connect the sensor unit 110 and the transmission unit 120 by using an electric contact makes it unnecessary to mount the transmission unit 120 on the sensor unit 110 in tight contact with each other at a predetermined connecting position. That is, it is possible to mount the transmission unit 120 on the sensor unit 110 with the simple lock portions.

As a consequence, the subject can easily attach and detach the transmission unit 120 to and from the sensor unit 110. That is, the subject can attach and detach the transmission unit 120 with ease. This allows the subject to easily detach the transmission unit 120 when taking a shower or bath, and eliminates the necessity to make the transmission unit 120 have a waterproof structure. It is therefore possible to implement the system at a low cost.

### [Second Embodiment]

The first embodiment is configured to make the transmission unit 120 supply all power necessary for measurement by the component measurement unit 115 without incorporating any battery in the sensor unit. However, the present invention is not limited to this, and may be configured to incorporate a battery in the sensor unit and make the battery supply power necessary for measurement by the component measurement unit 115.

### <1. Functional Arrangement of Bodily Fluid Component Measurement System>

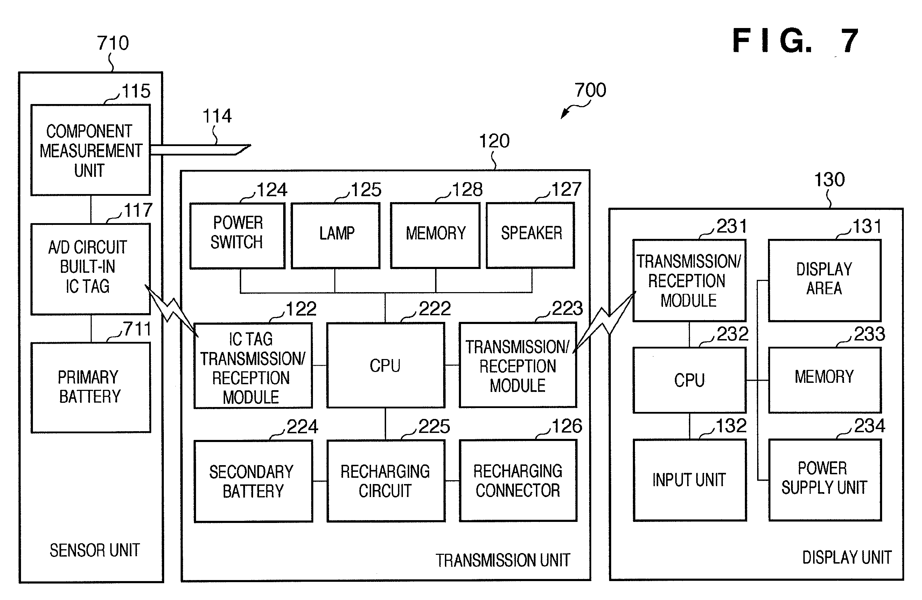

Fig. 7 is a block diagram showing the functional arrangement of a bodily fluid component measurement system 700 according to this embodiment. Note that the differences from the bodily fluid component measurement system 100 shown in Fig. 2 will be mainly described below.

Referring to Fig. 7, reference numeral 711 denotes a compact button type primary battery (for example, an alkali battery or lithium battery), which supplies power to a component measurement unit 115. An IC tag 117 is activated by using the electromotive force generated by the electromagnetic field generated in a transmission unit 120, and controls the component measurement unit 115 to operate by using power from the primary battery 711. The IC tag 117 also transmits, to the transmission unit 120, a digital measurement signal obtained by A/D-converting the measurement result from the component measurement unit 115.

As described above, in the bodily fluid component measurement system 700 according to this embodiment, the primary battery 711 is built in a sensor unit 710, and the component measurement unit 115 operates on the power supplied from the primary battery 711, thereby allowing the component measurement unit 115 to stably perform measurement. Note that the component measurement unit 115 transmits a measurement signal to the transmission unit 120 by using the electromotive force generated in the IC tag 117 or the power supplied from the primary battery 711.

### <2. Measurement Processing>

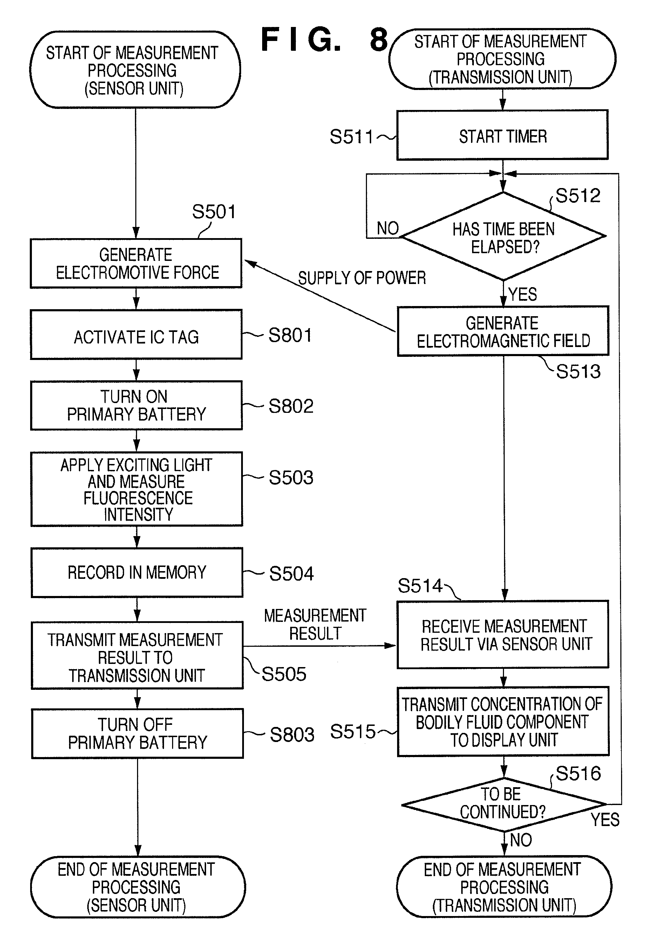

Measurement processing (step S305 in the first embodiment) in the bodily fluid component measurement system 700 will be described in detail next. Fig. 8 is a flowchart showing a detailed procedure for measurement processing (step S305) in the bodily fluid component measurement system 700.

Note that the processing from step S511 to step S516 is the same as that from step S511 to step S516 in Fig. 5 associated with the first embodiment, and hence a description of the processing will be omitted.

In step S513, an IC tag transmission/reception module 122 generates an electromagnetic field to generate an electromotive force in the IC tag 117 in step S501. With this operation, in step S801, the IC tag 117 is activated to control the primary battery 711 to supply power to the component measurement unit 115. With this operation, in step S802, the primary battery 711 starts to supply power to the component measurement unit 115 to activate the component measurement unit 115.

The processing from step S503 to step S505 is the same as that from step S503 to step S505 in Fig. 5, and hence a description of the processing will be omitted.

In step S803, the IC tag 117 controls the primary battery 711 to stop supplying power to the component measurement unit 115. With this operation, the primary battery 711 stops supplying power to the component measurement unit 115.

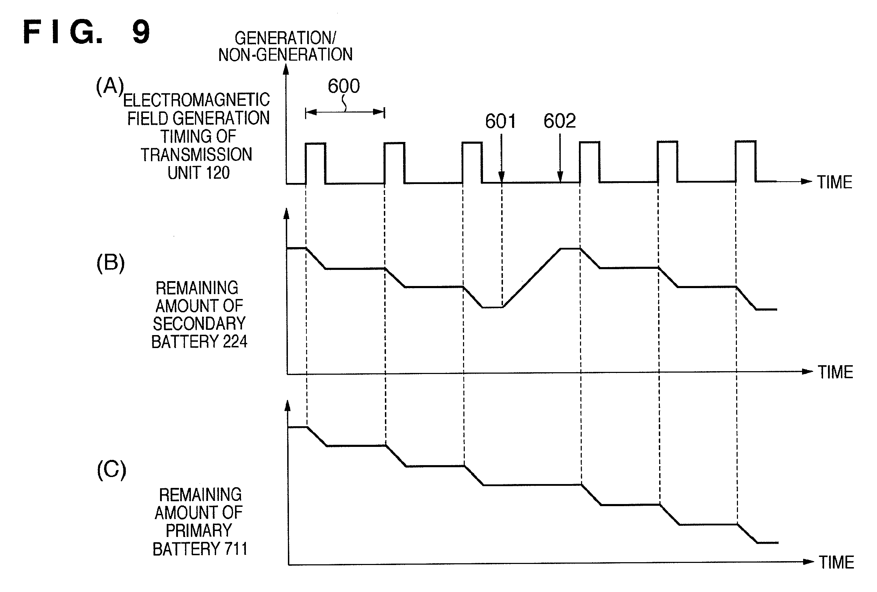

<3. Electromagnetic Field Generation Timing of Transmission Unit, Change in Remaining Amount of Secondary Battery, and Change in Remaining Amount of Primary Battery>
The electromagnetic field generation timing of the transmission unit 120, a change in the remaining amount of a secondary battery 224, and a change in the remaining amount of a primary battery 711 of a sensor unit 710 at the time of measurement processing by the bodily fluid component measurement system 700 will be described next. Fig. 9 is a schematic view showing the electromagnetic field generation timing of the transmission unit 120, a change in the remaining amount of the secondary battery 224, and a change in the remaining amount of the primary battery 711 of the sensor unit 710 at the time of measurement processing by the bodily fluid component measurement system 700.

As shown in Fig. 9, when measurement processing starts, the IC tag transmission/reception module 122 generates an electromagnetic field every predetermined period 600 to activate the IC tag 117. As a result, the remaining amount of the secondary battery 224 decreases every predetermined period 600. As the IC tag 117 is activated, the primary battery 711 supplies power to the component measurement unit 115. The component measurement unit 115 then performs measurement. As a consequence, the remaining amount of the primary battery 711 decreases every predetermined period 600.

Assume that after measurement was executed a plurality of number of times, the subject has temporarily detached the transmission unit 120 from the sensor unit 710 and performed recharging operation to take a shower or bath (timing 601).

With this operation, the remaining amount of the secondary battery 224 increases, and recharging completes at a timing 602. Subsequently, when the subject attaches the transmission unit 120 to the sensor unit 710 again and turns on the power supply, measurement processing starts again. The IC tag transmission/reception module 122 generates an electromagnetic field again every predetermined period 600 to activate the IC tag 117. As a result, the remaining amount of the secondary battery 224 in the transmission unit 120 decreases every predetermined period 600.

On the other hand, the primary battery 711 continuously decreases every predetermined period 600 without being recharged. The primary battery 711 therefore has a capacity corresponding to the upper limit of measurement frequency in the replacement cycle of the sensor unit 710.

As is obvious from the above description, the bodily fluid component measurement system 700 according to this embodiment is configured to perform supply of power and transmission of measurement signals between the sensor unit 710 and the transmission unit 120 by using the IC tag 117 and the IC tag transmission/reception module 122. In addition, this system is configured to incorporate the primary battery as a power supply in the sensor unit 110.

This makes it possible to obtain the same effects as those of the first embodiment and stably operate the component measurement unit.

### [Third Embodiment]

The first embodiment is configured to incorporate the primary battery in the sensor unit. The present invention is not limited to this, and may be configured to incorporate a rechargeable secondary battery in the sensor unit.

### <1. Functional Arrangement of Bodily Fluid Component Measurement System>

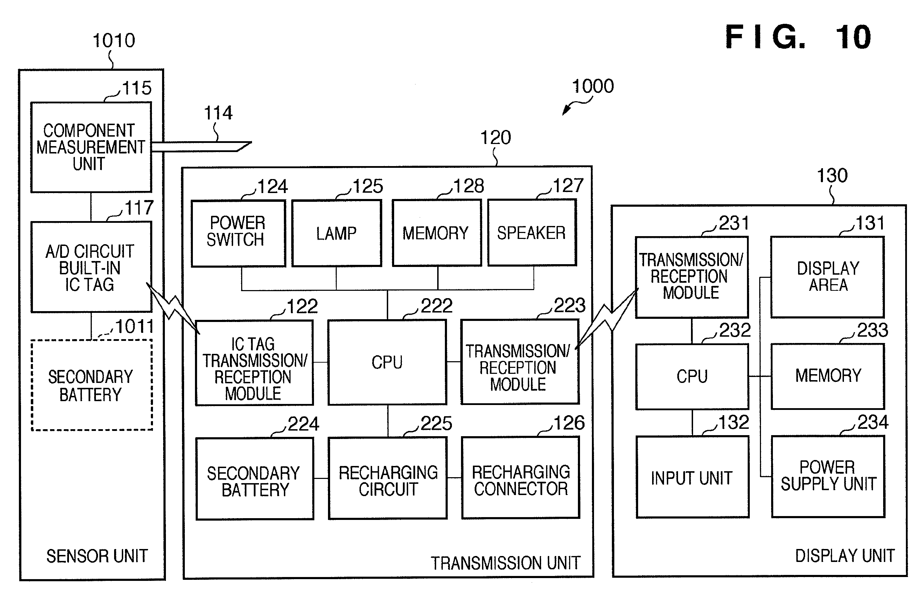

Fig. 10 is a block diagram showing the functional arrangement of a bodily fluid component measurement system 1000 according to this embodiment. Note the differences from the bodily fluid component measurement system 100 shown in Fig. 2 or the bodily fluid component measurement system 700 shown in Fig. 7 will be mainly described below.

Referring to Fig. 10, reference numeral 1011 denotes a compact button type secondary battery (for example, a lithium battery), which supplies power to a component measurement unit 115. An IC tag 117 is activated by using the electromotive force generated by the electromagnetic field generated in a transmission unit 120, and controls the component measurement unit 115 to operate on power from the secondary battery 1011. The IC tag 117 also transmits, to the transmission unit 120, a digital measurement signal obtained by A/D-converting the measurement result from the component measurement unit 115.

When the remaining amount of the secondary battery 1011 becomes equal to or less than a predetermined value, the secondary battery 1011 is recharged by the electromotive force generated by the electromagnetic field generated in the transmission unit 120.

As described above, the bodily fluid component measurement system 1000 according to this embodiment is configured to incorporate the secondary battery 1011 in a sensor unit 1010 and make the component measurement unit 115 operate on the power supplied from the secondary battery 1011. This makes it possible to stably perform measurement by the component measurement unit 115. In addition, when the remaining amount of the secondary battery 1011 becomes equal to or less than a predetermined value, the secondary battery can be recharged. This eliminate the chance of battery depletion in the sensor unit 1010 and allows this technique to be applied to a sensor which consumes larger power.

Note that when transmitting a measurement signal to the transmission unit 120, the component measurement unit 115 uses the electromotive force generated by the IC tag 117 or the power supplied from the secondary battery 1011.

### <2. Measurement Processing>

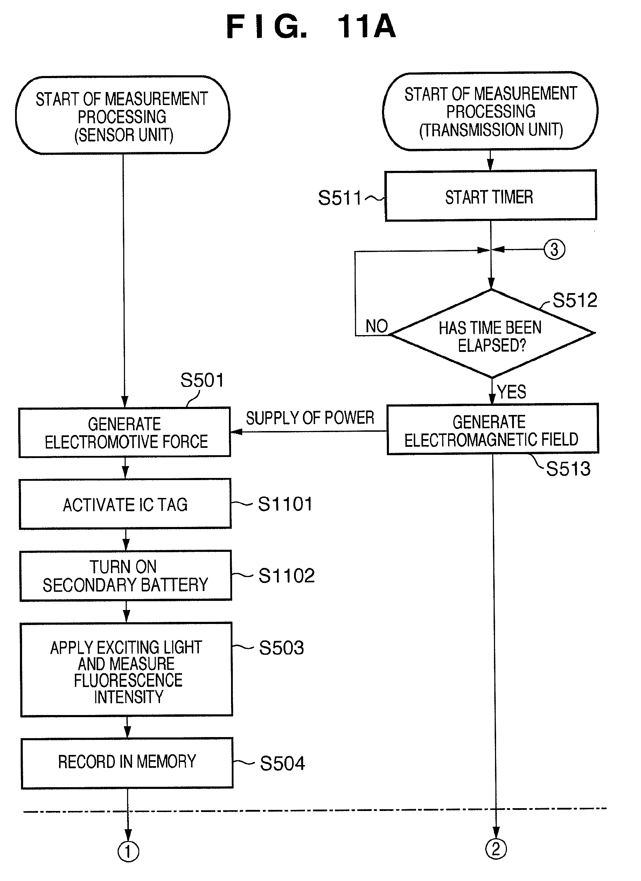

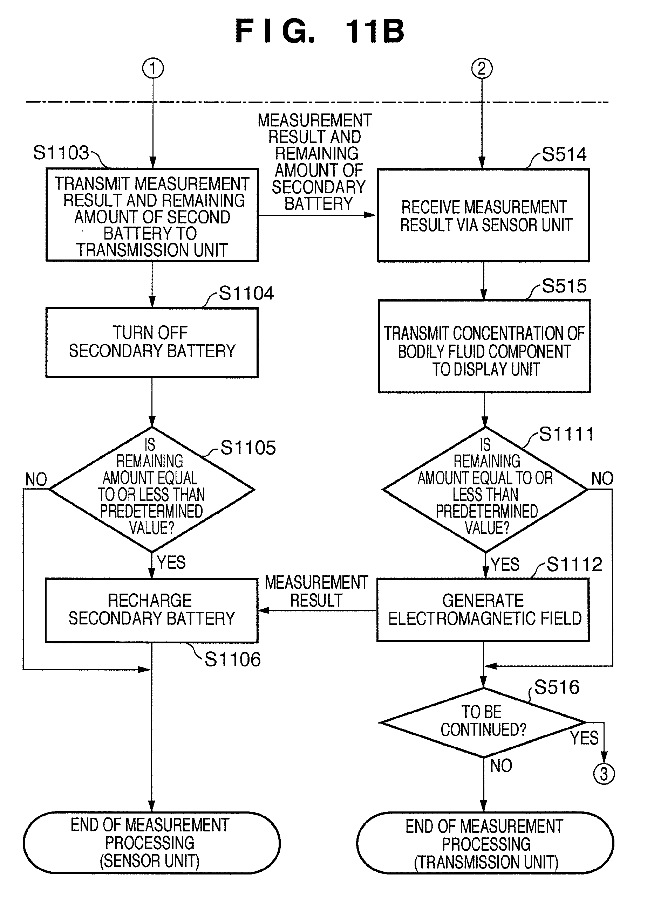

Measurement processing (step S305 in the first embodiment) in the bodily fluid component measurement system 1000 will be described in detail next. Figs. 11A and 11B are flowcharts showing a detailed procedure for measurement processing (step S305) in the bodily fluid component measurement system 1000.

Note that the processing from step S511 to step S515 is the same as that from step S511 to step S515 in Fig. 8 associated with the second embodiment, and hence a description of the processing will be omitted.

In step S513, an IC tag transmission/reception module 122 is activated to generate an electromotive force in the IC tag 117 in step S501. In step S1101, the IC tag 117 is activated to control the secondary battery 1011 to supply power to the component measurement unit 115. With this operation, in step S1102, the secondary battery 1011 starts to supply power to the component measurement unit 115 to activate the component measurement unit 115.

The processing from step S503 to step S504 is the same as that from step S503 to step S504 in Fig. 8, and hence a description of the processing will be omitted.

In step S1103, the IC tag 117 transmits, to the IC tag transmission/reception module 122, a measurement signal indicating a fluorescence intensity converted into a digital signal. The IC tag 117 also detects the remaining amount of the secondary battery 1011 and transmits the detection result as secondary battery remaining amount data to the IC tag transmission/reception module 122.

In step S1104, the IC tag 117 controls the secondary battery 1011 to stop supplying power to the component measurement unit 115. With this operation, the secondary battery 1011 stops supplying power to the component measurement unit 115.

Upon receiving the measurement signal transmitted by the imaging optical system 101, the transmission unit 120 determines in step S1111 whether the secondary battery remaining amount data transmitted by the sensor unit 1010 is equal to or less than a predetermined value. If the transmission unit 120 determines in step S1111 that the secondary battery remaining amount data is equal to or less than the predetermined value, the process advances to step S1112. If the transmission unit 120 determines that the secondary battery remaining amount data is not equal to or less than the predetermined value, the process advances to step S516 (since the processing in step S516 is the same as that in step S516 in Fig. 8, a description of the processing will be omitted).

In step S1112, the system drives the IC tag transmission/reception module 122. In step S1105, the electromagnetic field generated in the IC tag transmission/reception module 122 generates an electromotive force in the IC tag 117 (that is, the transmission unit 120 supplies power to the sensor unit 1010).

In step S1105, the IC tag 117 determines whether the secondary battery remaining amount data is equal to or less than the predetermined value. If the IC tag 117 determines in step S1105 that the secondary battery remaining amount data is equal to or less than the predetermined value, the process advances to step S1106, in which as the transmission unit 120 supplies power in step S1112, the IC tag 117 is activated and performs control to recharge the secondary battery 1011 with the generated electromotive force. With this operation, in step S1106, the secondary battery 1011 is recharged.

If the IC tag 117 determines in step S1105 that the secondary battery remaining amount data is equal to or less than the predetermined value, the system terminates the measurement processing.

### <3. Electromagnetic Field Generation Timing of Transmission Unit, Change in Remaining Amount of Secondary Battery, and Change in Remaining Amount of Secondary Battery of Sensor Unit>

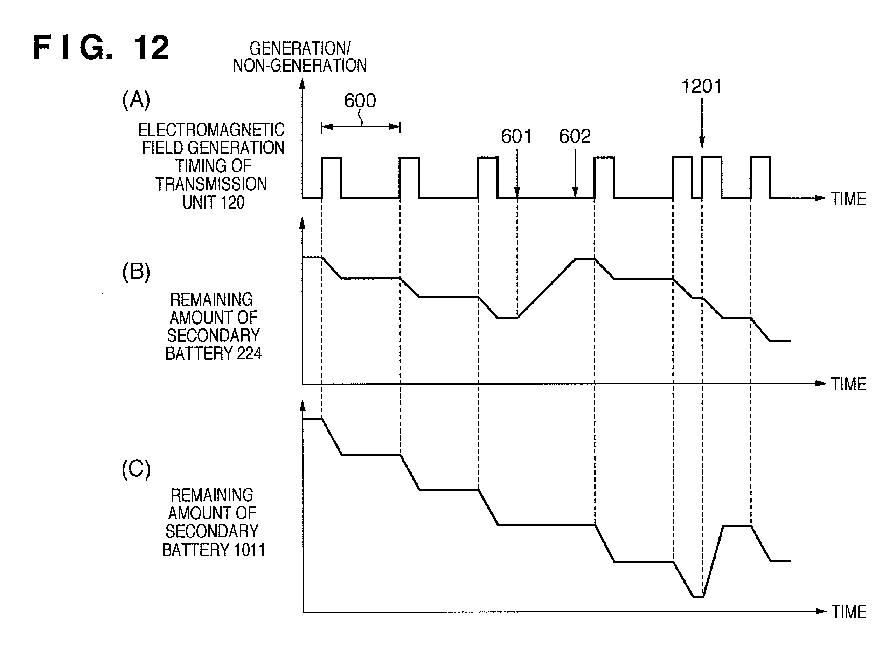

The electromagnetic field generation timing of the transmission unit 120, a change in the remaining amount of a secondary battery 224, and a change in the remaining amount of the secondary battery 1011 of the sensor unit 1010 at the time of measurement processing by the bodily fluid component measurement system 1000 will be described next. Fig. 12 is a schematic view showing the electromagnetic field generation timing of the transmission unit 120, a change in the remaining amount of the secondary battery 224, and a change in the remaining amount of the secondary battery 1011 of the sensor unit 1010 at the time of measurement processing by the bodily fluid component measurement system 1000.

As shown in Fig. 12, when the measurement processing starts, the IC tag transmission/reception module 122 generates an electromagnetic field every predetermined period 600 to drive the IC tag 117. As a result, the remaining amount of the secondary battery 224 decreases every predetermined period 600. In addition, as the IC tag 117 is activated, the secondary battery 1011 starts to supply power to the component measurement unit 115, thereby performing measurement. As a result, the remaining amount of the secondary battery 1011 decreases every predetermined period 600.

Assume that after measurement was executed a plurality of number of times, the subject has temporarily detached the transmission unit 120 from the sensor unit 1010 and performed recharging operation to take a shower or bath (timing 601).

With this operation, the remaining amount of the secondary battery 224 increases, and recharging completes at a timing 602. Subsequently, when the subject attaches the transmission unit 120 to the sensor unit 1010 again and turns on the power supply, measurement processing starts again. The IC tag transmission/reception module 122 generates an electromagnetic field again every predetermined period 600 to activate the IC tag 117. As a result, the remaining amount of the secondary battery 224 decreases every predetermined period 600.

On the other hand, the remaining amount of the secondary battery 1011 decreases every predetermined period 600. If the system determines that the remaining amount is equal to or less than a predetermined value, the transmission unit 120 drives the IC tag transmission/reception module 122 to generate an electromagnetic field for recharging the secondary battery 1011 upon changing the frequency of electromagnetic field generation (timing 1201). Assume however that in this case, the frequency of measurement is not changed.

With this operation, the remaining amount of the secondary battery 224 of the transmission unit 120 decreases, and the remaining amount of the secondary battery 1011 of the sensor unit 1010 increases. That is, the secondary battery 1011 in the sensor unit 1010 can be a capacitance that does not depend on the frequency of measurement in the replacement cycle of the sensor unit 1010.

As is obvious from the above description, the bodily fluid component measurement system 1000 according to this embodiment is configured to perform supply of power for activating the IC tag, supply of power for recharging the secondary battery, and transmission of measurement signals between the sensor unit 1010 and the transmission unit 120 by using the IC tag 117 and the IC tag transmission/reception module 122. In addition, this system is configured to incorporate a secondary battery as a power supply for measurement in the sensor unit 1010.

This makes it possible to obtain the same effects as those of the first embodiment and to make the component measurement unit stably operate. In addition, it is possible to avoid power depletion in the sensor unit.

Note that this embodiment is configured to recharge a secondary battery after measurement of a biological component and transmission of a measurement signal. However, the embodiment may be configured to measure a biological component and transmit a measurement signal after recharging the secondary battery. This makes it possible to reliably supply power for measurement because of recharging operation immediately before the measurement.

### [Fourth Embodiment]

The third embodiment is configured to incorporate the rechargeable secondary battery in the sensor unit. However, the present invention is not limited to this and may be configured to provide a capacitor in the sensor unit and recharge the capacitor.

### <1. Functional Arrangement of Bodily Fluid Component Measurement System>

The functional arrangement of the bodily fluid component measurement system according to this embodiment is the same as that described with reference to Fig. 2 in the first embodiment, and hence a description of the functional arrangement will be omitted. Assume that in the bodily fluid component measurement system shown in Fig. 2, an IC tag 117 provided in a sensor unit 110 further incorporates a capacitor. The bodily fluid component measurement system including the IC tag 117 incorporating a capacitor will be described as a bodily fluid component measurement system 1300.

The IC tag 117 incorporating the capacitor is activated by the electromotive force generated by the electromagnetic field generated in a transmission unit 120. The capacitor stores the electromotive force. A component measurement unit 115 is then controlled to operate on the power stored in the capacitor.

The bodily fluid component measurement system 1300 according to this embodiment is configured to incorporate the capacitor in the sensor unit 110 and make the component measurement unit 115 operate on the power stored in the capacitor. It is possible to make the component measurement unit 115 stably perform measurement.

### <2. Measurement Processing>

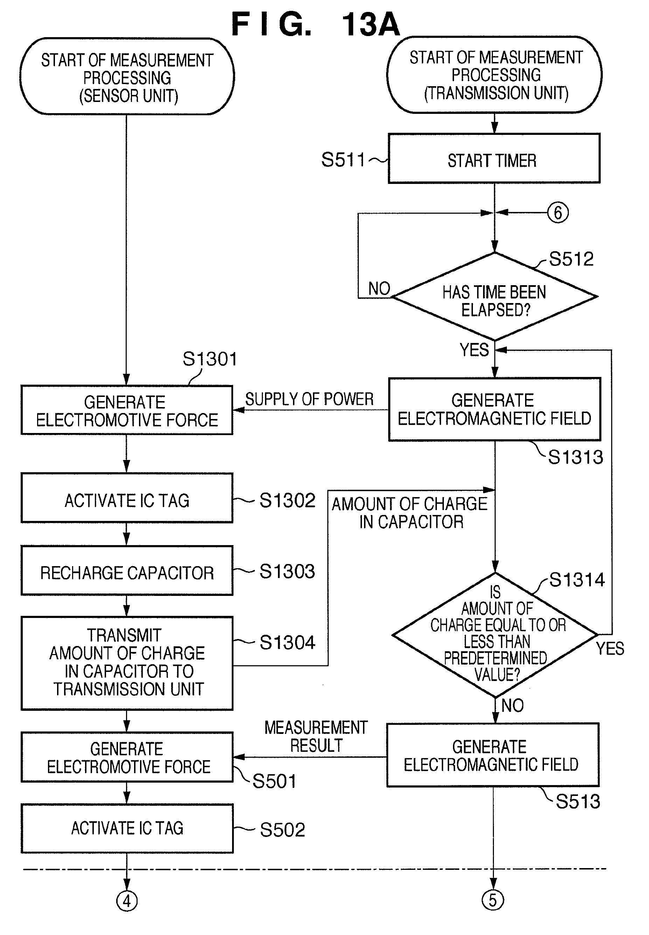

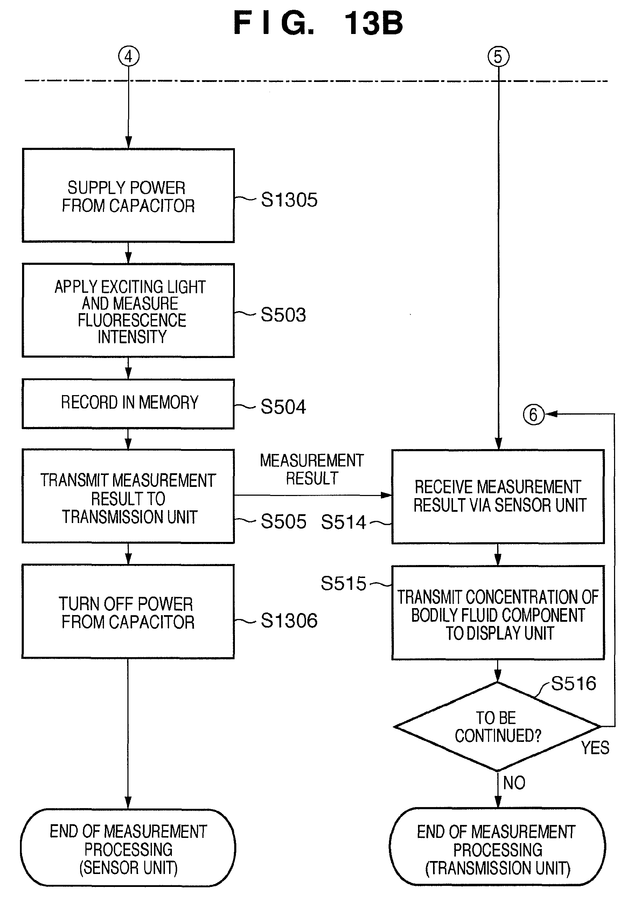

Measurement processing (step S305 in the first embodiment) in the bodily fluid component measurement system 1300 will be described in detail next. Figs. 13A and 13B are flowcharts showing a detailed procedure for measurement processing (step S305) in the bodily fluid component measurement system 1300.

Note that the processing from step S511 to step S512 is the same as that from step S511 to step S512 in Fig. 5 associated with the first embodiment, and hence a description of the processing will be omitted.

An IC tag transmission/reception module 122 is activated in step S1313 to generate an electromotive force in the IC tag 117 in step S1301. This activates the IC tag 117 in step S1302. In step S1303, part of the generated electromotive force is charged in the capacitor. In step S1304, the amount of charge stored in the capacitor is transmitted to the transmission unit 120.

The transmission unit 120 determines, based on the amount of charge in the capacitor transmitted from the sensor unit 110, in step S1314 whether the charge of amount in the capacitor is equal to or less than a predetermined value. If the transmission unit 120 determines in step S1314 that the amount of charge is equal to or less than the predetermined value, the process returns to step S1313 to repeat the above processing until the amount of charge in the capacitor exceeds the predetermined value. If the transmission unit 120 determines in step S1314 that the amount of charge is not equal to or less than the predetermined value, the process advances to step S513.

Since the processing from step S513 to step S516 is the same as that from step S513 to step S516 in Fig. 5 associated with the first embodiment, and hence a description of the processing will be omitted.

The IC tag transmission/reception module 122 is activated in step S513 to generate an electromotive force in the IC tag 117 in step S501. This activates the IC tag 117 in step S502. In step S1305, the capacitor starts to supply power to the component measurement unit 115. As a result, the component measurement unit 115 is activated.

Since the processing from step S503 to step S505 is the same as that from step S503 to step S505 in Fig. 5, and hence a description of the processing will be omitted.

In step 1306, the IC tag 117 controls the capacitor to supply power to the component measurement unit 115. With this operation, the capacitor stops supplying power to the component measurement unit 115.

### <3. Electromagnetic Field Generation Timing of Transmission Unit, Change in Remaining Amount of Secondary Battery, and Change in Remaining Amount of Capacitor of Sensor Unit>

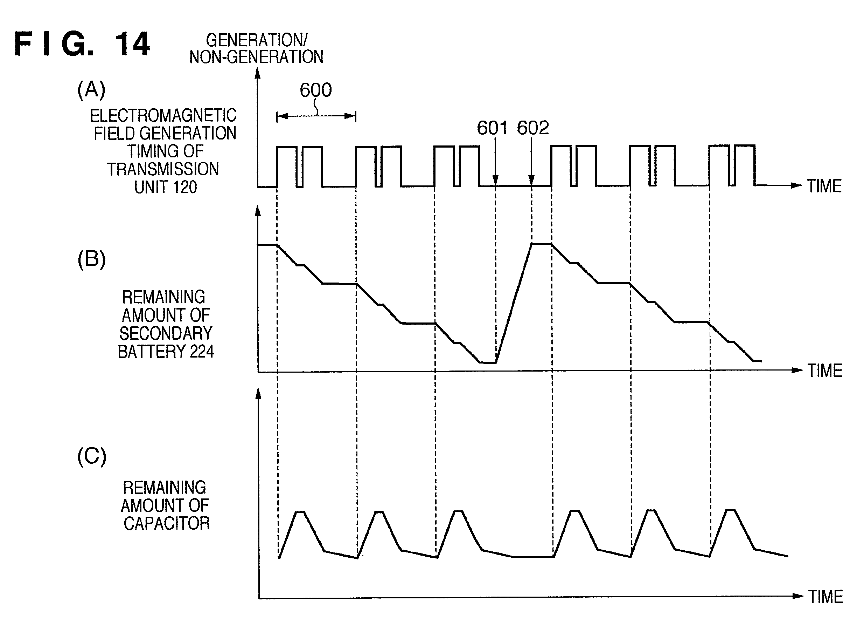

The electromagnetic field generation timing of the transmission unit 120, a change in the remaining amount of a secondary battery 224, and a change in the remaining amount of the capacitor of the sensor unit 110 at the time of measurement processing by the bodily fluid component measurement system 1300 will be described next. Fig. 14 is a schematic view showing the electromagnetic field generation timing of the transmission unit 120, a change in the remaining amount of the secondary battery 224, and a change in the remaining amount of the capacitor of the sensor unit 110 at the time of measurement processing by the bodily fluid component measurement system 1300.

As shown in Fig. 14, when measurement processing starts, the IC tag transmission/reception module 122 generates an electromagnetic field continuously two times every predetermined period 600 to activate the IC tag 117 in each operation. As a result, the remaining amount of the secondary battery 224 decreases every predetermined period 600. In addition, upon the first activation of the IC tag 117, the capacitor is recharged. Upon the second activation of the IC tag 117, the capacitor starts supplying power to the component measurement unit 115, thereby performing measurement. This reduces the remaining amount of the capacitor. After the measurement is complete, the remaining amount of the capacitor gradually decreases due to self-discharge.

Assume that after measurement was executed a plurality of number of times, the subject has temporarily detached the transmission unit 120 from the sensor unit 110 and performed recharging operation to take a shower or bath (timing 601).

With this operation, the remaining amount of the secondary battery 224 increases to complete charging operation at a timing 602. Subsequently, when the subject attaches the transmission unit 120 to the sensor unit 110 and turns on the power supply, the measurement processing is resumed, and the IC tag transmission/reception module 122 generates an electromagnetic field again continuously two times every predetermined period 600 to activate the IC tag 117 in each operation. As a result, the remaining amount of the secondary battery 224 decreases every predetermined period 600. On the other hand, the capacitor is repeatedly recharged and discharged every predetermined period 600.

As is obvious from the above description, the bodily fluid component measurement system 1300 according to this embodiment is configured to perform supply of power for activating the IC tag, supply of power for recharging the capacitor, and transmission of measurement signals between the sensor unit 110 and transmission unit 120 by using the IC tag 117 and the IC tag transmission/reception module 122. This system is also configured to incorporate the capacitor as a power supply for measurement in the sensor unit 110.

This makes it possible to obtain the same effects as those of the first embodiment described above and make the component measurement unit stably operate. In addition, it is possible to avoid power depletion in the sensor unit.

### [Fifth Embodiment]

The first to fourth embodiments are configured to calculate calibration value data in the transmission unit 120 by inputting the blood glucose level obtained by measurement in advance by SMBG via the display unit 130. However, the present invention is not limited to this. For example, the present invention may be configured to provide an IC tag for a blood glucose meter used for SMBG and directly input from the blood glucose meter to a blood glucose level for calibration to a transmission unit 120 from the blood glucose meter via an IC tag transmission/reception module 122.

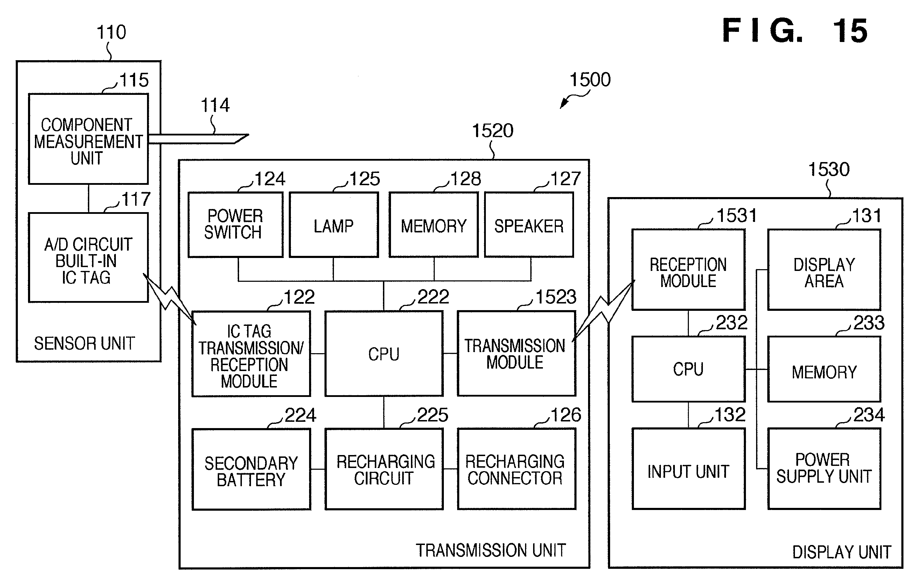

Fig. 15 is a block diagram showing the functional arrangement of a bodily fluid component measurement system 1500 which directly receives a blood glucose level for calibration from an external blood glucose meter (not shown) via a transmission unit 1520. As shown in Fig. 15, this system is configured to directly transmit a blood glucose level from the external blood glucose meter to the IC tag transmission/reception module 122 when calculating a blood glucose level. This eliminates the necessity to transmit information for calculating calibration value data via a display unit 1530. For this reason, the display unit 1530 is provided with a reception module 1531 instead of a transmission/reception module.

Likewise, since it is not necessary to receive information for calculating calibration value data from the display unit 1530, the transmission unit 1520 is provided with a transmission module 1523 instead of a transmission/reception module. This can construct a simpler system.

Although the first to fifth embodiments have been described above with reference to Figs. 1 to 15, the present invention is not limited to these embodiments. For example, in the first to fifth embodiments, the display unit is provided as a display device placed at a position away from the subject. However, the present invention is not limited to this. The display unit may be provided as a display device which can be attached and detached to and from the subject.

More specifically, the display unit may be configured to be attached to the wrist or ankle of the subject or to hang from the neck of the subject. Alternatively, the display unit may be configured to be attached to the waist of the subject. This allows the subject to quickly check a calculated blood glucose level.

The first to fifth embodiments have exemplified the case in which the electromagnetic induction scheme is used as a data transfer scheme between the IC tag 117 and the IC tag transmission/reception module 122. However, the present invention is not limited to this, and may use an electromagnetic wave scheme. In addition, the embodiments have exemplified the case in which the type of tag is a passive tag. However, it is possible to use other types of tags. That is, the present invention may use a tag based on any type of communication scheme as long as it is a tag based on a near or neighboring field noncontact communication scheme used as an RFID tag.

In addition, the first to fifth embodiments have exemplified the case in which the fluorescence sensor designed to measure a fluorescence intensity is used as a blood glucose level measurement method. However, the present invention is not limited to this, and may use other measurement methods (for example, a GOD sensor).

Furthermore, in the first to fifth embodiments, an interstitial fluid is set as a measurement target, and a blood glucose level is calculated based on the amount of glucose contained in the interstitial fluid. However, the present invention is not limited to this, and may measure any kind of bodily fluid component by using a known measurement method as long as the bodily fluid component allows to calculate its concentration.

For example, in a case of a patient with hypertension, it is necessary to monitor bodily fluid components such as salt and cholesterol, and hence the present invention may be configured to calculate their concentrations. Alternatively, the present invention may be configured to calculate the concentrations of various kinds of components contained in blood, such as mineral, protein, and fat.

### [Sixth Embodiment]

The first to fifth embodiments are configured to provide the needle 114 connected to the component measurement unit 115 on the central portion of the bottom surface of the sensor unit 110 to puncture the skin in a direction almost perpendicular to the bottom surface. However, the present invention is not limited to this, and may be configured to make a needle obliquely puncture the skin while making the needle detachable.

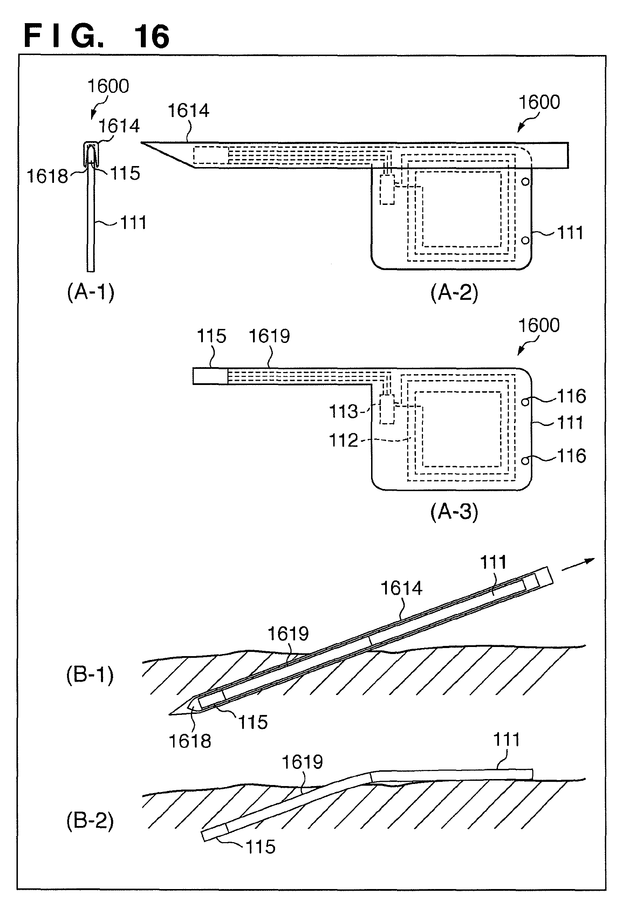

Fig. 16 shows the outer arrangement of a sensor unit 1600 (which can obliquely puncture the subject) and the puncturing state in this embodiment. In Fig. 16, (A-1) is a side view of the sensor unit 1600, (A-2) is a plan view of the sensor unit 1600 to which an introduction needle 1614 is attached, and (A-3) is a plan view of the sensor unit 1600 from which the introduction needle 1614 is detached.

As indicated by (A-3), the sensor unit 1600 is provided with a puncturing portion 1619 extending from a side surface end portion of a main body portion 111 almost parallel to the bottom surface of the sensor unit 1600. A component measurement unit 115 is mounted on the distal end of the puncturing portion 1619. Note that the main body portion 111 and the puncturing portion 1619 are formed from one member and have flexibility.

The main body portion 111 incorporates an IC tag 117 including an antenna 112, an IC chip 113, and an A/D conversion circuit (not shown). The main body portion 111 is configured to operate on the electromotive force as power which is generated by the electromagnetic field generated in a transmission unit 120, control measurement by the component measurement unit 115, store a measurement result in a memory in the IC chip 113, and transmit the result to the transmission unit 120. The upper surface of the main body portion 111 is provided with a lock portion 116 and is configured to lock to the upper surface side of the transmission unit 120.

In addition, as indicated by (A-2), the introduction needle 1614 is detachably mounted on the side surface of the main body portion 111, the component measurement unit 115, and the puncturing portion 1619. The sensor unit 1600 in this embodiment is configured such that when the introduction needle 1614 punctures the skin of the subject, the component measurement unit 115 and the puncturing portion 1619 disposed in the introduction needle 1614 are guided to under the skin of the subject. This brings the component measurement unit 115 into contact with a bodily fluid.

As indicated by (A-1), the introduction needle 1614 mounted to cover the component measurement unit 115 and the puncturing portion 1619 is provided with a notched portion 1618 throughout the total length. This allows the introduction needle 1614 to be detached after it guides the component measurement unit 115 to under the skin of the subject by puncturing the skin.

(B-1) indicates a state in which the component measurement unit 115 punctures the skin of the subject with the introduction needle 1614. As indicated by (B-1), when the introduction needle 1614 obliquely punctures the skin of the subject, the component measurement unit 115 is guided to under the skin of the subject.

As described above, since the introduction needle 1614 is provided with the notched portion 1618, it is possible to remove only the introduction needle 1614 by pulling out the introduction needle 1614 in the arrow direction while the introduction needle 1614 is kept punctured in the subject.

(B-2) is a view showing a state in which only the introduction needle 1614 is removed after it punctures the skin of the subject. Note that the main body portion 111 and the puncturing portion 1619 are made of a resin such as polyimide. After the introduction needle 1614 is removed, the puncturing portion 1619 bends from a portion which is not inserted into the skin, and the main body portion 111 is bonded along the skin of the subject with an adhesive (not shown) provided on the lower surface of the main body portion 111. Note that since the IC chip 113 and the A/D conversion circuit (not shown) have some thickness, they are disposed away from the portion through which the introduction needle 1614 passes.

The bodily fluid component measurement systems according to the first to fifth embodiments can be configured to include the sensor unit shown in Fig. 16 (the sensor unit configured to obliquely puncture the skin and make the needle detachable).

### [Seventh Embodiment]

The sixth embodiment is configured to make the puncturing portion extend from a side surface end portion of the main body. However, the present invention is not limited to this, and may be configured to make the puncturing portion extend from a side surface central portion of the main body portion.

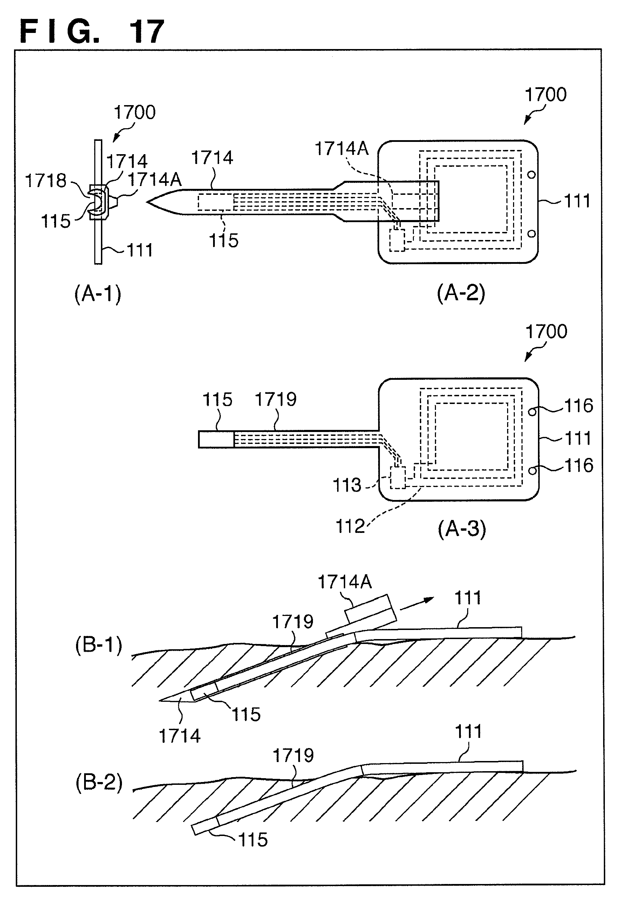

Fig. 17 shows the outer arrangement of a sensor unit 1700 (which can obliquely puncture the subject) and the puncturing state in this embodiment. In Fig. 17, (A-1) is a side view of the sensor unit 1700, (A-2) is a plan view of the sensor unit 1700 to which an introduction needle 1714 is attached, and (A-3) is a plan view of the sensor unit 1700 from which the introduction needle 1714 is removed.

As indicated by (A-3), the sensor unit 1700 is provided with a puncturing portion 1719 extending from a side surface central portion of a main body portion 111 almost parallel to the bottom surface of the sensor unit 1700. A component measurement unit 115 is mounted on the distal end of the puncturing portion 1719. The main body portion 111 and the puncturing portion 1719 are formed from one member and have flexibility.

The main body portion 111 incorporates an IC tag 117 including an antenna 112, an IC chip 113, and an A/D conversion circuit (not shown). The main body portion 111 is configured to operate on the electromotive force as power which is generated by the electromagnetic field generated in a transmission unit 120, control measurement by the component measurement unit 115, store a measurement result in a memory in the IC chip 113, and transmit the result to the transmission unit 120. The upper surface of the main body portion 111 is provided with a lock portion 116 and is configured to lock to the upper surface side of the transmission unit 120.

In addition, as indicated by (A-2), the introduction needle 1714 is detachably mounted on the component measurement unit 115 and the puncturing portion 1719. The sensor unit 1700 in this embodiment is configured such that when the introduction needle 1714 punctures the skin of the subject, the component measurement unit 115 and the puncturing portion 1719 disposed in the introduction needle 1714 are guided to under the skin of the subject. This brings the component measurement unit 115 into contact with a bodily fluid.

As indicated by (A-1), the introduction needle 1714 mounted to cover the component measurement unit 115 and the puncturing portion 1719 is provided with a notched portion 1718 throughout the total length. This makes it possible to remove the introduction needle 1714 by pulling out a knob portion 1714A after the introduction needle 1714 punctures the skin of the subject to guide the component measurement unit 115 to under the skin of the subject.

(B-1) indicates a state in which the component measurement unit 115 punctures the skin of the subject with the introduction needle 1714. As indicated by (B-1), when the introduction needle 1714 obliquely punctures the skin of the subject, the component measurement unit 115 is guided to under the skin.

Note that the main body portion 111 and the puncturing portion 1719 are made of a resin such as polyimide. After the introduction needle 1714 obliquely punctures the skin of the subject, the puncturing portion 1719 bends from a portion which is not inserted into the skin, and the main body portion 111 is bonded along the skin of the subject with an adhesive (not shown) provided on the lower surface of the main body portion 111. Thereafter, the introduction needle 1714 is pulled out of the skin, and the placement of the sensor unit 1700 is complete.

As described above, since the introduction needle 1714 is provided with the notched portion 1718, it is possible to remove only the introduction needle 1714 by pulling the knob portion 1714A and pulling out the introduction needle 1714 in the arrow direction while the introduction needle 1714 is kept punctured in the subject.

(B-2) is a view showing a state in which only the introduction needle 1714 is removed after it punctures the skin of the subject.

The bodily fluid component measurement system according to the first to sixth embodiments can be configured to include the sensor unit shown in Fig. 17 (the sensor unit configured to obliquely puncture the skin and make the needle detachable).

The present invention is not limited to the above-described embodiments, and various changes and modifications can be made within the spirit and scope of the present invention. Therefore, to apprise the public of the scope of the present invention, the following claims are made.

This application claims the benefit of Japanese Patent Application No. 2010-081405, filed March 31, 2010 which is hereby incorporated by reference herein in its entirety.

## Claims

1. A bodily fluid component measurement system **characterized by** comprising:
a sensor unit which is indwelled in the skin of a subject and connected to a bodily fluid in the subject to measure a predetermined bodily fluid component; and
a transmission unit which is detachably attached to said sensor unit and transmits, to a display device, a measurement signal obtained by said sensor unit or a calculation result calculated as a concentration of the bodily fluid component in a bodily fluid of the same type as or different type from the bodily fluid based on the measurement signal,
**characterized in that** said sensor unit measures the bodily fluid component and transmits the measurement signal to said transmission unit by being activated by electromagnetic induction caused by an electromagnetic field generated by said transmission unit.

2. The bodily fluid component measurement system according to claim 1, **characterized in that** said sensor unit incorporates an IC tag, and
when said transmission unit generates an electromagnetic field, said sensor unit measures the bodily fluid component and transmits the measurement signal to said transmission unit by using an electromotive force generated in said IC tag.

3. The bodily fluid component measurement system according to claim 1, **characterized in that** said sensor unit incorporates an IC tag and a primary battery, and
when said transmission unit generates an electromagnetic field, said sensor unit measures the bodily fluid component by using power supplied by said primary battery, and transmits the measurement signal to said transmission unit by using an electromotive force generated in said IC tag or power supplied from said primary battery.

4. The bodily fluid component measurement system according to claim 1, **characterized in that** said sensor unit incorporates an IC tag and a secondary battery or a capacitor, and
when said transmission unit generates an electromagnetic field, said sensor unit recharges said secondary battery or said capacitor by using an electromotive force generated in said IC tag, measures the bodily fluid component by using power supplied from said secondary battery or said capacitor, and transmits the measurement signal to said transmission unit by using power supplied from said secondary battery or said capacitor.

5. The bodily fluid component measurement system according to claim 4, **characterized in that** said IC tag detects a remaining amount of said secondary battery or said capacitor, and transmits the detection result to said transmission unit.

6. The bodily fluid component measurement system according to claim 5, **characterized in that** said transmission unit changes a frequency of generation of the electromagnetic field in accordance with the detection result.

7. The bodily fluid component measurement system according to claim 1, **characterized in that** when receiving a measurement signal from said sensor unit, said transmission unit or said display device calculates a concentration of the bodily fluid component in a bodily fluid of the same type as or different from the bodily fluid by using the measurement signal and calibration value data.

8. The bodily fluid component measurement system according to claim 7, **characterized in that** the calibration value data is calculated based on a concentration of the bodily fluid component in the same type as or different type from the bodily fluid measured by an external device and a measurement signal measured by said sensor unit, and the concentration of the bodily fluid component measured by the external device is transmitted from the external device to said display device, transmitted to said transmission unit via said display device, or directly transmitted to said transmission unit.

9. The bodily fluid component measurement system according to claim 1, **characterized in that** said transmission unit is configured to operate on a rechargeable secondary battery.
